# EUROPEAN PATENT SPECIFICATION

(11) **EP 3 645 094 B1**
(45) Date of publication and mention of the grant of the patent: **12.10.2022**
(21) Application number: 17807856.4
(22) Date of filing: 30.11.2017
(51) Int. Cl.: A61M 16/00, A61M 16/20, A61M 16/08, A61M 16/10, A61M 16/12

(54) **RESUSCITATION BAG WITH PEP EXHAUST VALVE COMPATIBLE WITH THORACIC COMPRESSIONS**
BEATMUNGSBEUTEL MIT EINEM MIT THORAXKOMPRESSIONEN KOMPATIBLEN PEP-AUSLASSVENTIL
BALLON DE RÉANIMATION COMPORTANT UNE SOUPAPE D'ÉCHAPPEMENT EN PEP COMPATIBLE AVEC DES COMPRESSIONS THORACIQUES

(30) Priority: 27.06.2017 US 201762525399 P
(43) Date of publication of application: 06.05.2020
(73) Proprietor: Air Liquide Santé International, 75007 Paris (FR); Air Liquide Medical Systems, 92160 Antony (FR)
(72) Inventor: BOULANGER, Thierry, Media, PA 19063 (US); RICHARD, Jean-Christophe, 92160 Antony (FR); RIGOLLOT, Marceau, 92160 Antony (FR); GINER, Jean-Marc, 92250 La Garenne Colombes (FR)
(74) Representative: Air Liquide
(86) International application number: PCT/EP2017/080969
(87) International publication number: WO 2019/001751

(56) References cited:
- EP-A1- 1 512 426
- WO-A1-2009/047763
- DE-U1- 9 208 860
- US-A- 2 834 339
- US-A- 4 367 767
- US-A- 5 427 091
- US-A- 5 884 623
- US-A- 6 102 038
- US-A1- 2004 007 235
- US-A1- 2004 206 354
- US-B1- 6 425 393

## Description

### Cross Reference to Related Application

This application claims the benefit of priority to U.S. Provisional Patent Application No. 62/525,399, filed June 27, 2017.

### Background

The present invention relates to an artificial respiration device, namely an artificial resuscitation bag that can be used for resuscitating a person, i.e. a patient, in state of cardiac arrest, and an installation comprising such an artificial resuscitation bag for resuscitating a person in state of cardiac arrest.

Cardiac arrest is a condition affecting hundreds of thousand people every year with a very poor prognosis.

One of the main lifesaving actions is to apply thoracic compressions or 'TCs' along with brief intervals of lung ventilation with a resuscitation bag. TCs are successive compressions and decompressions exerted on the thoracic cage of the person, i.e. the patient, in cardiac arrest. TCs aim at partially restoring inhalation and exhalation phases and therefore gas exchanges in the lungs, as well as promoting or restoring blood circulation toward the organs and especially the brain of the patient.

As these compressions and decompressions only mobilize small volumes of gas in and out of the patient's airways, it is advocated to perform regularly further gas insufflations to bring fresh O₂-containing gas into the lungs thereby enhancing the gas exchanges.

Usually, fresh O₂-containing gas is delivered by a resuscitation bag linked with an oxygen source and connected to the patient through a respiratory interface, typically a facial mask, a laryngeal mask, or an endotracheal tube.

To date, it is recommended to interpose 2 insufflations every 30 chest compressions, whereas the ideal rate of compressions, according to international guidelines, is between 100 and 120 compressions per minute (c/min).

However, several studies have shown that it is difficult for rescuers to correctly perform the resuscitation sequence and that the interruptions of TCs to initiate the insufflations with a resuscitation bag are often too long and deleterious with respect to the patient's outcome, as rapidly affecting the hemodynamic, i.e., in other words, offsetting the benefits of the TCs themselves.

Some respiratory assistance devices have been proposed for overcoming the drawbacks associated with resuscitation bags. Among them, the most popular are Continuous Positive Airway Pressure apparatus, also called "CPAP systems" or "CPAP devices", that rely on an oxygen containing-gas supply, at a pressure above 1 atm, for creating a continuous positive pressure at the patient's airways depending on the continuous flow of oxygen (e.g. the higher the oxygen flow, the higher the positive pressure).

During thoracic compressions/decompressions, small volumes are flowing in and out of the patient's airways at a positive pressure which helps keep the alveoli of the lungs open thereby promoting and/or enhancing gas exchanges. In addition, the positive pressure creates a resistance to gas expulsion during the TC phases, which improves the energy transmission to the heart thereby promoting a better cardiac output.

However, if these CPAP systems have demonstrated to be beneficial over basic TCs, e.g. without an extra respiratory assistance device, and could represent an interesting alternative, there is still room for improvement as small volumes are still mobilized during the TCs and intermittent insufflations with a resuscitation bag would be beneficial to bring further fresh O₂-containing gas into the lungs and thereby improve CO₂ clearance.

Unfortunately, such CPAP systems cannot function with current resuscitation bags without threatening the patient's life, due to serious adverse events that can be caused by the design of the CPAP systems themselves (especially from the continuous flow of oxygen that is supposed to set the positive pressure of the CPAP system).

WO 2009/047763 A1, US 2004/007235 A1, US 5427091 A, US 2004/206354 A1, DE 9208860 U1, US 6425393 B1 and US 2834339 A disclose bag systems for use in connection with resuscitation procedures.

### Summary

A main goal of the present invention is to fix the problem encountered with current resuscitation bags, in particular to provide an improved resuscitation bag allowing continuous TCs and, when required, enabling insufflations of given volumes of fresh O₂-containing gas, while keeping a continuous positive pressure of gas into the patient's lungs, without the need of any CPAP systems.

A solution according to the present invention concerns an artificial resuscitation bag according to claim 1. The dependent claims define preferred embodiments of the invention. An artificial resuscitation bag according to the invention comprises:
- a deformable bag comprising a gas inlet and a gas outlet,
- a gas reservoir comprising an outlet orifice,
- a first conduit element fluidly connected to the outlet orifice of the gas reservoir and to the gas inlet of the deformable bag,
- a first one-way admission valve arranged in the first conduit element and fluidly communicating with the ambient atmosphere for allowing ambient air to enter into the first conduit element, and
- a second one-way valve arranged in the first conduit element between the first one-way admission valve and the gas inlet of the deformable bag for allowing the gas to travel only from the first conduit element to the deformable bag,
and further comprises:
- a first PEP exhaust valve arranged in the first conduit element and fluidly communicating with the ambient atmosphere for venting gas to the atmosphere when the gas pressure, into the first conduit element, exceeds a given threshold, and
- a gas conduit arranged in fluid communication with the gas outlet of the deformable bag,
- an overpressure valve arranged in the gas conduit,
- a third one-way valve arranged in said gas conduit downstream of the overpressure valve, and
- a gas delivery conduit in fluid communication with the gas conduct for conveying at least part of the gas circulating into the gas conduct to a patient interface,

wherein the first PEP exhaust valve comprises a spring and a membrane, said spring applying a constant force on the membrane corresponding to the threshold pressure, the opening pressure of said PEP exhaust valve being of between 1 cmH2O and 30 cmH2O, and
wherein the artificial resuscitation bag further comprises:
   - a pneumatic control valve arranged in the gas conduit downstream of the third one-way valve, and
   - a derivation conduct having a first end fluidly connected to the gas conduit between the gas outlet of the deformable bag and the overpressure valve, and a second end fluidly connected to the pneumatic control valve, the pneumatic control valve further comprising an inner compartment, the second end of the derivation conduct being fluidly connected to said inner compartment of the pneumatic control valve.

Depending on the embodiment, an artificial resuscitation bag according to the present invention can comprise of one or several of the following additional features:
- the opening pressure of PEP exhaust valve is of at least 5 cmH₂O.
- the first PEP exhaust valve comprises an inlet port in fluid communication with the first conduit.
- the first conduit element comprises an oxygen entry arranged between the outlet orifice of the gas reservoir and the second one-way valve.
- the first conduit element comprises an inner passage for the gas.
- the pneumatic control valve comprises a deformable membrane.
- the patient interface comprises of a respiratory mask or a tracheal cannula.
- the gas conduit conveys at least a part of the gas exiting the deformable bag through the gas outlet.
- the overpressure valve is configured to vent to the atmosphere at least part of the gas present in the gas conduit, when the gas pressure in the gas conduit exceeds a given value.
- the artificial resuscitation bag further comprises of a second one-way valve arranged in a conduit in fluid communication with the gas inlet of the deformable bag.
- the third one-way valve is configured for allowing a circulation of gas in the gas conduit only in the direction from the deformable bag toward the pneumatic control valve.
- it further comprises an oxygen line fluidly connected to the first conduit.
- it further comprises an oxygen distribution system comprising a gas distributor and a by-pass line connected to said gas distributor.
- the gas distributor is arranged on the oxygen line.
- the by-pass line is fluidly connected to the gas distributor and to the patient interface.

Further, the present invention also concerns an installation for resuscitating a person in state of cardiac arrest comprising:
- an artificial resuscitation bag according to the present invention, and
- an O₂ source fluidly connected to the artificial resuscitation bag by means of an oxygen line, for providing oxygen to said artificial resuscitation bag.

### Brief Description of the Drawings

For a further understanding of the nature and objects for the present invention, reference should be made to the following detailed description, taken in conjunction with the accompanying drawings, in which like elements are given the same or analogous reference numbers and wherein:
- Figure 1 represents an embodiment of the resuscitation bag according to the prior art.
- Figure 2 illustrates an embodiment of the resuscitation bag according to the prior art.
- Figure 3A illustrates an embodiment of the resuscitation bag according to the prior art.
- Figure 3B illustrates an embodiment of the resuscitation bag according to the prior art.
- Figure 4 illustrates an embodiment of the resuscitation bag according to the prior art.
- Figure 5 illustrates an embodiment of the resuscitation bag according to the prior art.
- Figure 6 illustrates an embodiment of the resuscitation bag according to the prior art.
- Figure 7A illustrates an embodiment of the resuscitation bag according to the present invention.
- Figure 7B illustrates an embodiment of the resuscitation bag according to the present invention.
- Figure 7C illustrates an embodiment of the resuscitation bag according to the present invention.
- Figure 8 illustrates an embodiment of the resuscitation bag according to the present invention.
- Figure 9 illustrates an embodiment of the resuscitation bag according to the present invention.
- Figure 10 illustrates an embodiment of the resuscitation bag according to the present invention.
- Figure 11 illustrates an embodiment of the resuscitation bag according to the present invention.
- Figure 12 is another embodiment of the resuscitation bag, not according to the present invention.
- Figure 13A illustrates an embodiment of a pneumatic control valve of a resuscitation bag according to an embodiment of the present invention.
- Figure 13B illustrates an embodiment of a pneumatic control valve of a resuscitation bag according to an embodiment of the present invention.

### Description of Preferred Embodiments

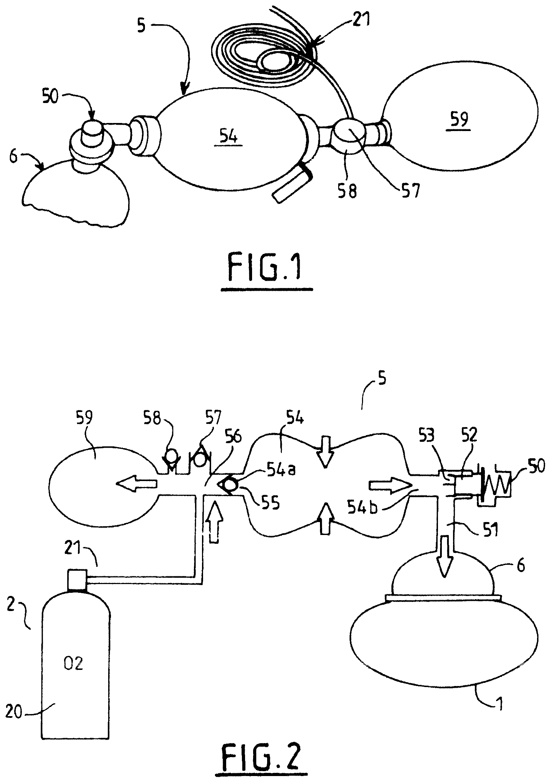

Figures 1 and 2 show a commercially available resuscitation bag 5 comprising of a respiratory interface **6** for feeding a gas to a patient, typically a respiratory mask, a flexible bag **54,** and a valve element **50** for diverting the gas in and out of the patient, during insufflation and exsufflation phases, and a source of an oxygen-containing gas **2,** such as or including a gas cylinder **20** containing oxygen, which is delivered during insufflation phases.

The flexible bag **54** is filled with fresh gas formed by a mixture of oxygen provided by an oxygen line **21** connected to the oxygen source **2** (cf. Figure 2), typically a medical grade oxygen cylinder **20,** and ambient air provided by an admission valve **57** in fluid communication with the ambient atmosphere.

A supplementary gas reservoir **59** can be added to increase the availability of oxygen. Further, a first exhaust valve **58** is provided for venting gas in the case of overpressure.

In Figure 2, a patient **1** is connected to the resuscitation bag **5,** via a respiratory interface **6,** e.g. a facial mask, a laryngeal mask or similar.

The oxygen source **2,** typically a cylinder **20** of medical grade oxygen, is fluidly connected via an oxygen line or tubing **21** and a first conduit element **56,** to the flexible bag **54,** the tubing **21** being fluidly connected to the first conduit element **56.** The first conduit element **56** is further fluidly communicating with the inlet orifice **54A** of the flexible bag **54.**

When the operator squeezes the flexible bag **54** to perform an insufflation of gas to the patient **1,** the flow of gas exiting the flexible bag **54** through its outlet orifice **54B** travels to the patient **1** into the lumen of a second conduit **51** that is fluidly connected to the respiratory interface **6,** such as a facial mask. At the same time, the flow of gas exiting the flexible bag **54** occludes the exhalation port **52** of a third exhaust valve **53** that is arranged in the second conduit **51,** i.e. downstream of gas bag **54,** as shown in Figure 2.

This generates positive pressure which, as a result, forces second one-way valve **55** arranged upstream of the bag **54** to close thereby preventing the gas of bag **54** to flow backward, i.e. in the first conduit element **56,** and to escape via the first exhaust valve **58.** Meanwhile, the flow of oxygen travelling in tubing **21** enters into the first conduit element **56** and fills the supplementary reservoir **59** that is fluidly connected to first conduit element **56.**

Due to the slightly positive pressure in first conduit element **56,** the air admission valve **57** is closed. In the case where the reservoir **59** becomes over-distended by the entering flow of gas, a pressure increase will occur in first conduit element **56** and the gas in excess will be vented to the ambient atmosphere by the first exhaust valve **58.** The opening pressure of the first exhaust valve **58** is close to 0, but slightly positive due to mechanical frictions.

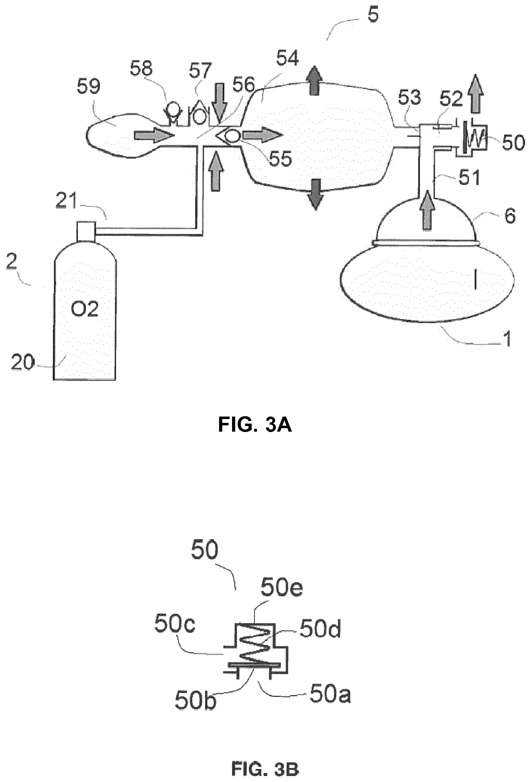

Figure 3A shows an expiration phase of the commercially available resuscitation bag **5** of Figures 1 and 2, when the operator has stopped squeezing the bag **54,** which bag **54** enters in an expansion phase due to a negative pressure that holds back the third exhaust valve **53** thereby opening the exhalation port **52.** The volume of gas accumulated in the patient's airways during the preceding inspiratory phase will travel through interface **6** and second conduit **51** before being vented to ambient atmosphere through the exhalation port **52.**

The resuscitation bag **5** can also include a PEP valve **50** that creates a positive expiratory pressure, during exhalation phases, thereby helping keeping open the alveoli of the lungs of patient **1.**

As detailed in Figure 3B, such a PEP valve **50** typically comprises a spring **50d** arranged in a housing **50e,** which applies a constant force on a membrane **50b.** The gas pressure in the PEP valve inlet port **50a,** that is in fluid communication with exhalation port **52** and that applies on said membrane **50b,** has to be sufficiently high for exerting a force greater than the load of the spring **50d** for displacing the membrane **50b** backward and opening a fluidic pathway between the inlet port **50a** and an outlet port **50c** of the PEP valve inlet port **50a.** The fluidic pathway allows the gas pressure to escape through the outlet port **50c,** thereby allowing an expiration of gas by the patient **1.** It is possible to set the load of spring **50d** to different expiratory pressures, such as expiratory pressures corresponding to 5 cmH₂O,10 cmH₂O,or 20 cmH₂O.

At the same time, the negative pressure generated in bag **54** will open the second one-way valve **55** that will: i) direct the gas flow from tubing **21** into bag **54** via conduit **56,** ii) empty reservoir **59** into bag **54** via conduit **56,** and iii) open the air admission valve **57** thereby allowing ambient air entering successively into conduit **56** and bag **54,** as shown in Figure 3A.

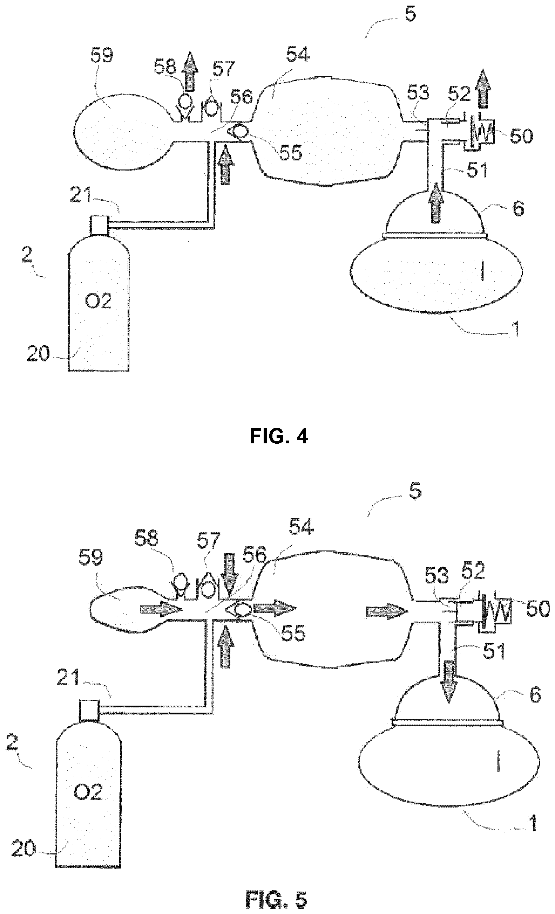

Further, Figures 4-6 show a sequence of thoracic compressions (TC) in association with the resuscitation bag **5** of Fig. 1, 2 and 3A.

In Figure 4, the resuscitation bag **5** is represented in its "rest" state, i.e. not active state, for example as it is before being used. The gas bag **54** and reservoir **59** are filled with gas and ready for an insufflation. The oxygen flowing from cylinder **20** and tubing **21** enters conduit **56** and is vented to the atmosphere through the first exhaust valve **58.** When the bag **54** is in its "rest" state, the operator usually starts to exert thoracic compressions or TCs on the patient **1.** Due to the TCs, the third exhaust valve **53** is pushed back, i.e. closed, thereby occluding the fluidic pathway **52** between gas bag **54** and second conduit **51.** Indeed, a TC expels a small volume of gas from the patient's airways which travels backwardly through second conduit **51,** exhaust port **52,** and PEP valve **50.** Actually, PEP valve **50** creates a resistance force against expired gases, which will promote or restore blood circulation in the patient's body.

When a TC is relaxed, the patient **1** enters decompression and the airway pressure becomes negative as shown in Figure 5. The negative pressure closes PEP valve **50,** i.e. occlude the fluidic passage between ports **50a** and **50c** (cf. Figure 3B), and air is delivered by bag **54,** thereby pushing the third exhaust valve **53** toward the exhaust port **52** for creating a fluidic passage between said gas bag **54** and conduit **51.**

Meanwhile, the second one-way valve **55** allows: i) a first flow of gas, e.g. oxygen, to travel in tubing **21** and conduit **56,** and ii) a second flow of gas to exit reservoir **59** and to travel in conduit **56.**

Further, a third flow of gas, i.e. air, is allowed to penetrate into conduit **56** via the admission valve **57,** i.e. another one-way valve. These three flows of gas enter into bag **54** thereby filling said bag **54.**

However, with such architecture, several problems exist. For instance, the pressure in the patient's airways when the TC is relaxed will be equal to 0, i.e. not positive. This is clearly an issue as for providing efficient TCs, a positive pressure, such as 5 cm H₂O, is mandatory to force the alveoli of the patient to open and to improve gas exchanges.

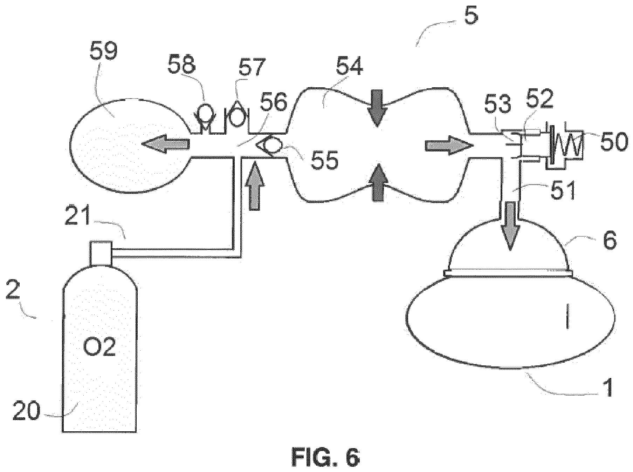

As shown in Figure 6, when the operator performs an insufflation as described earlier, if a TC occurs during this insufflation phase, third exhaust valve **53** and second one-way valve **55** will prevent any gas exhaust. This constitutes a risk for the patient **1** as an over-pressure will appear, which can be deleterious for the lungs of the patient **1.**

As shown in Figures 1-6, artificial resuscitation bags of the prior art do not allow to simultaneously performing safe and effective TCs and gas insufflations with the resuscitation bag. Indeed, with such known resuscitation bags, it is impossible, on the one hand, to provide TCs during insufflations phases without risking over-pressures in the lungs and, on the other hand, keep a positive pressure during decompression phases, which negatively impacts gas exchanges and outcomes for the patient.

The present invention proposes an artificial resuscitation bag **5** that can overcome the above issues.

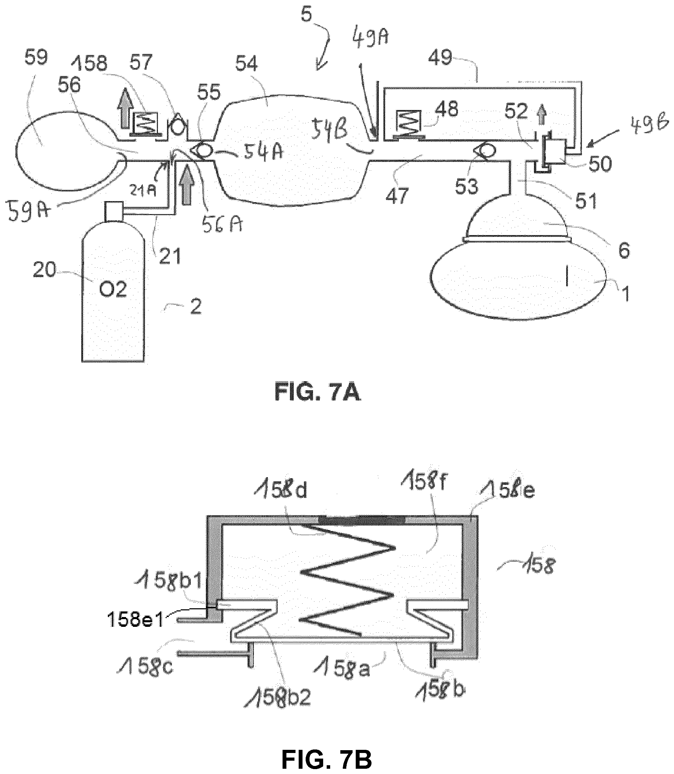

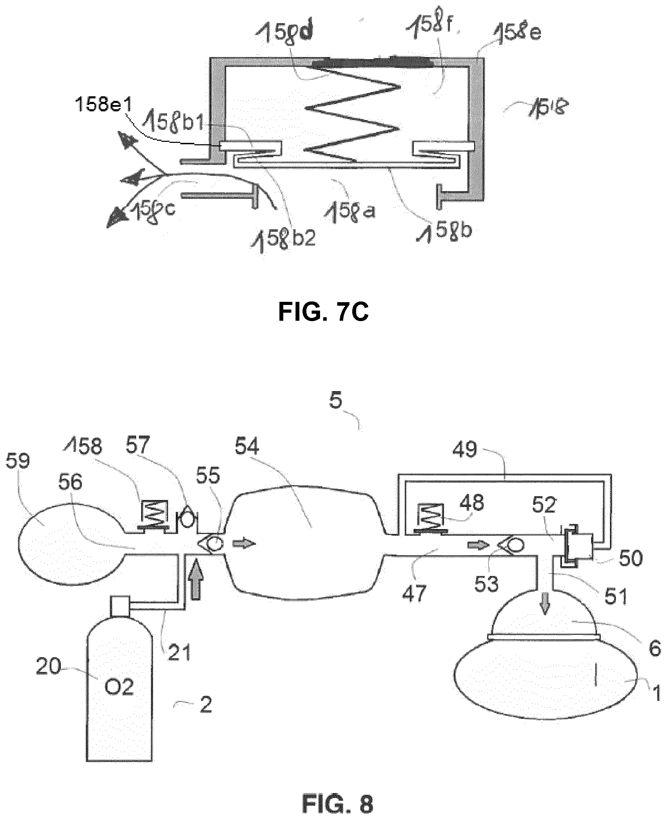

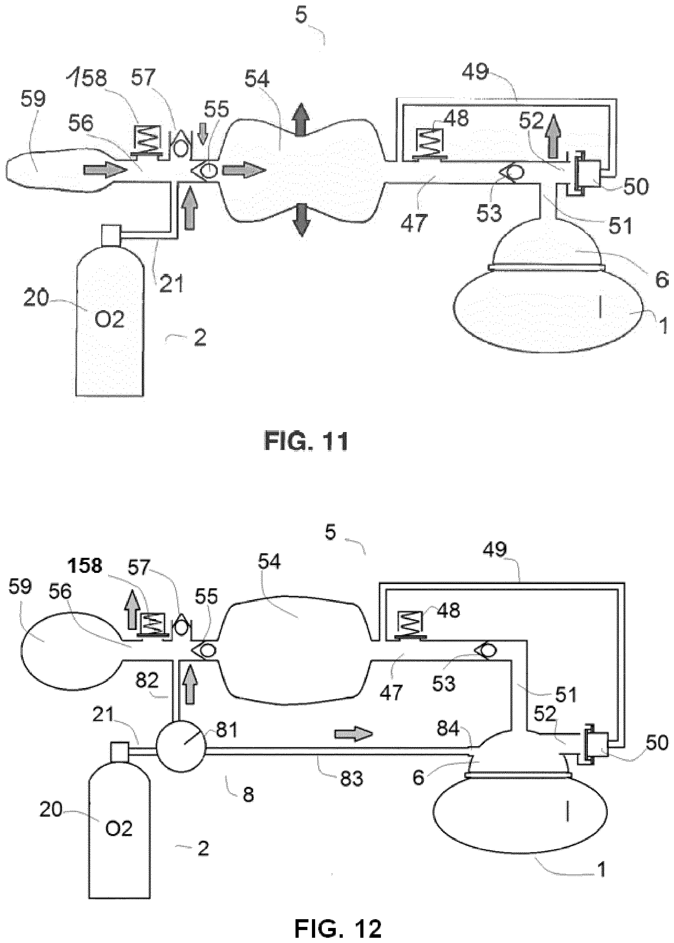

A first embodiment of an artificial resuscitation bag **5** according to the present invention is shown in Figures 7-11, whereas a second embodiment of an artificial resuscitation bag **5,** not according to the present invention is shown in Figure 12.

Figure 7A shows a first embodiment of a resuscitation bag **5** according to the present invention, allowing TCs to be performed while insufflating gas, and further keeping the patient's airways at a positive pressure level, i.e. greater than 0.

The artificial resuscitation bag **5** of Figures 7-12 has roughly the same architecture as the bag of Figure 1-6. It comprises a deformable bag **54** comprising a gas inlet **54A** and a gas outlet **54B,** a gas reservoir **59** comprising an outlet orifice **59A,** a first conduit element **56** fluidly connected to the outlet orifice **59A** of the gas reservoir **59** and to the gas inlet **54A** of the deformable bag **54,** a first one-way admission valve **57** arranged in the first conduit element **56** and fluidly communicating with the ambient atmosphere for allowing ambient air to enter into the first conduit element **56,** and a second one-way valve **55** arranged in the first conduit element **56** between the first one-way admission valve **57** and the gas inlet **54A** of the deformable bag **54** for allowing gas to travel only from the first conduit element **56** to the deformable bag **54.**

Further, the artificial resuscitation bag **5** of Figure 7A also comprises an overpressure valve **48,** also called "PPEAK valve", and a third one-way valve **53** arranged in the conduit **47** that is in fluid communication with the outlet **54B** of the deformable bag **54.**

The third one-way valve **53** prevents the gas to circulate backward in the conduit **47,** i.e. in the direction of the deformable bag **54,** whereas as the overpressure valve **48** is used for venting to the atmosphere any excess of pressure in the conduit **47,** between the deformable bag **54** and the third one-way valve **53.**

Furthermore, according to the present invention, the artificial resuscitation bag **5** of Figure 7A also comprises a first PEP exhaust valve **158** (PEP = Positive Expiration Pressure) arranged in the first conduit element **56** that fluidly communicates with the ambient atmosphere for venting gas to the atmosphere, when the gas pressure, into the first conduit element **56,** exceeds a given threshold between 1 cmH₂O and 30 cmH₂O, for instance a threshold pressure of about 5 cmH₂O.

In other words, the first exhaust valve **58** of Figures 1-6 has been replaced by the first PEP exhaust valve **158.**

Figure 7B shows a detailed embodiment of the first PEP exhaust valve **158.** It comprises a spring **158d** arranged in a housing **158e,** which applies a constant force on a membrane **158b** that corresponds to the threshold pressure of for instance about 5 cmH₂O.

The gas pressure in the inlet port **158a** of the first PEP exhaust valve **158,** that is in fluid communication with the first conduit element **56,** and that applies on said membrane **158b,** has to be sufficiently high for exerting a force greater than the load of the spring **158d** for displacing the membrane **158b** backward and opening a fluidic pathway between the inlet port **158a** and an outlet port **158c** of the first PEP exhaust valve **158,** i.e. a force greater than 5 cmH₂O for instance. This allows an excessive gas pressure in the first conduit element **56** to escape to the atmosphere through the outlet port **158c** of the first PEP exhaust valve **158.**

The load of spring **158d** has to be set at a desired threshold pressure, i.e. a given expiratory pressure, of between 1 cmH₂O and 30 cmH₂O, such as expiratory pressures corresponding to 5 cmH₂O,10 cmH₂O,20 cmH₂O or 30 cmH₂O.

The deformable membrane **158b** is tightly attached by its lips **158b1** to one or several grooves **158e1** arranged in the rigid structure forming the control valve housing **158e** of the first PEP exhaust valve **158.** A deformable portion **158b2** of membrane **158b** helps membrane **158b** moving forward or backward, depending on the pressure conditions.

At rest, membrane **158b** of the first PEP exhaust valve **158** prevents a fluidic connection between the inlet conduit **158a** and the outlet conduit **158c,** as shown in Figure 7B, due to the force exerted by load spring **158d** on membrane **158b.**

Figure 7C shows the first PEP exhaust valve **158** in its open position, when the gas pressure exceeds the threshold pressure level so that spring **158d** is compressed, thereby allowing gas to escape to the atmosphere through the outlet port **158c** of the first PEP exhaust valve **158.**

In Figure 7A, the resuscitation bag **5** is in an initial state or "rest" state in case of a thoracic compression. The gas reservoir **59** is filled with oxygen, the oxygen being provided by the O₂ source **2,** namely the cylinder **20** delivering oxygen to reservoir **59** via an oxygen-conveying tubing **21** and first conduit element **56.** The oxygen-conveying tubing **21** delivers oxygen to the first conduit element **56** through an oxygen entry **56A.**

The first PEP exhaust valve **158** is opened and vents the excess of gas to the atmosphere as the gas pressure exceeds the opening threshold pressure of the first PEP exhaust valve **158** that is set at 5 cmH₂O for example. This positive pressure keeps the first one-way valve **57** closed. This pressure will be equalized in all the parts behind the second one-way valve **55,** i.e. into bag **54** and subsequent components, such as conduits **47, 51** and **52.**

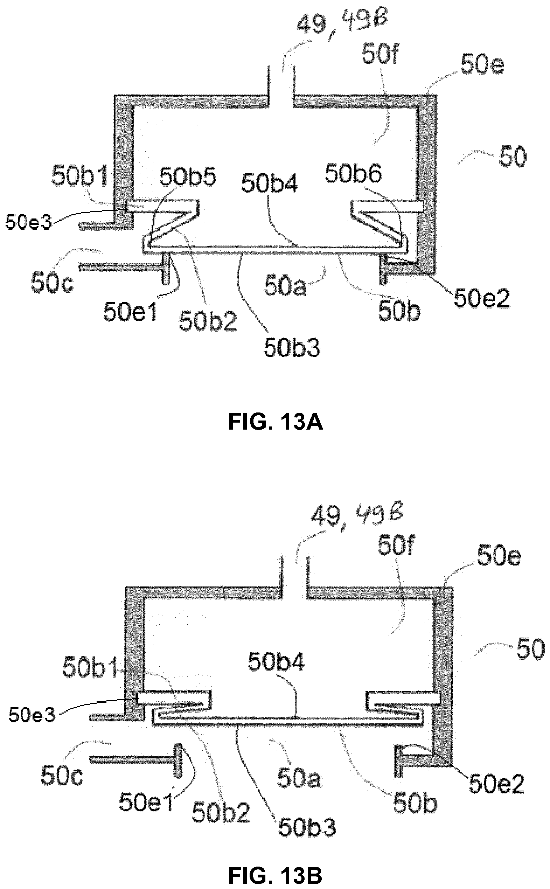

Further, the artificial resuscitation bag **5** of Figure 7A further comprises a pneumatic control valve **50** working in differential mode as shown in Figures 13A and 13B. The pneumatic control valve **50** comprises a deformable membrane **50b** that is tightly attached by its lips **50b1** to one or several grooves **50e3** in a rigid structure **50e,** which forms the pneumatic control valve **50** housing. A deformable portion **50b2** of membrane **50b** helps this membrane **50b** move forward or backward, depending on the conditions. At rest, this membrane **50b** prevents a fluidic connection between the inlet conduit **50a** and outlet conduit **50c,** as illustrated in Figure 13A.

This is due to the fact that membrane **50b** lays on edges **50e1** and **50e2** at rest, occluding inlet conduit **50a,** and further a surface area difference exists between inner side **50b4** and outer side **50b3** of membrane **50b.** Indeed, the inner side **50b4** of membrane **50b** is delimited by extremity points **50b5** and **50b6,** whereas the outer side of the membrane is defined as the diameter of inlet conduit **50a,** delimited by edges **50e1** and **50e2.** As a consequence, the surface of inner side **50b4** of membrane **50b** is greater than the surface of outer side **50b3** of membrane **50b.** Considering equal pressure on both sides of membrane **50b,** a positive force gradient from inner side **50b4** to outer side **50b3** is created. The mechanical strength of membrane **50b** laying on edges **50e1** and **50e2** and the positive force gradient generated by the surface difference between inner side **50b4** and outer side **50b3** of membrane **50b** will define an opening pressure threshold in inlet **50a** which will move membrane **50b** backward to allow a fluidic connection between inlet **50a** and outlet **50c,** as shown in Figure 13B. Depending on the size and characteristic of membrane **50b,** an opening pressure as low as 5mm H₂O can be set.

The pneumatic control valve **50** of Figures 13A and 13B further comprises a chamber **50f** which is fluidically connected to a derivation conduct **49** comprising a first end **49A** fluidly connected to the gas conduit **47,** between the gas outlet **54B** of the deformable bag **54** and the overpressure valve **48,** and a second end **49B** fluidly connected to the inner compartment **50f** of the pneumatic valve **50,** as shown in Figure 7A. Should the derivation conduct **49** provide a positive pressure, this pressure would add a force on top of the opening pressure defined above which will in turn make it harder to open the fluidic connection between inlet **50a** and outlet **50c,** unless the pressure at inlet **50a** follows the increase of pressure in chamber **50f,** offsetting its effect.

As shown in Figure 7A, at the very onset of a TC, the pressure in conduits **47** and **51,** in derivation conduct **49** and consequently in chamber **50f** of the pneumatic control valve **50** are equal and set to the PEP exhaust valve **158** value, e.g. 5cmH2O. This means that only the opening pressure of pneumatic control valve **50** will oppose the rise of pressure resulting from the TC. Following the example set above, as soon as the pressure exceeds 5.5 cmH₂O in second conduit **51** (e.g. sum of initial PEP exhaust valve **158** value of 5 cmH₂O plus the opening pressure of 5 mmH₂O), thus closing the third one-way valve **53,** the pneumatic control valve **50** will open to make a fluidic connection between inlet **50a** and outlet **50c,** allowing the volume expelled by the patient **1** to travel through interface **6,** conduits **51** and **52,** inlet **50a** and exhaust port, or outlet **50c.**

After a TC, follows a decompression phase as shown in Figure 8. The pressure in the patient's airways suddenly decreases to potentially subatmospheric pressures. As a consequence, the flow of oxygen in first conduit element **56,** coming from tubing **21,** will be directed to the patient **1** to offset this decrease in pressure, opening the second one-way valve **55** and the third one-way valve **53,** also called "exhalation valve", and traveling through flexible bag **54** and conduits **47, 51** and interface **6.** In addition, the pressure across the pneumatic control valve **50,** which is between derivation conduct **49** and therefore chamber **50f,** and conduit **52** and therefore inlet **50a** will be close to 0 and as a result the pneumatic control valve **50** will be closed.

As a result a direct fluidic pathway will be created between the oxygen supply in tubing **21** and patient **1.** However, the first PEP exhaust valve **158** will avoid any pressure greater that e.g. 5 cmH₂O in this fluidic pathway and will open, if necessary, to keep the pressure steady. In other words, in the phase of decompression, the patient **1** pressure airway will be kept close to e.g. 5 cm H₂O which will keep the alveoli open and enhance gas exchange.

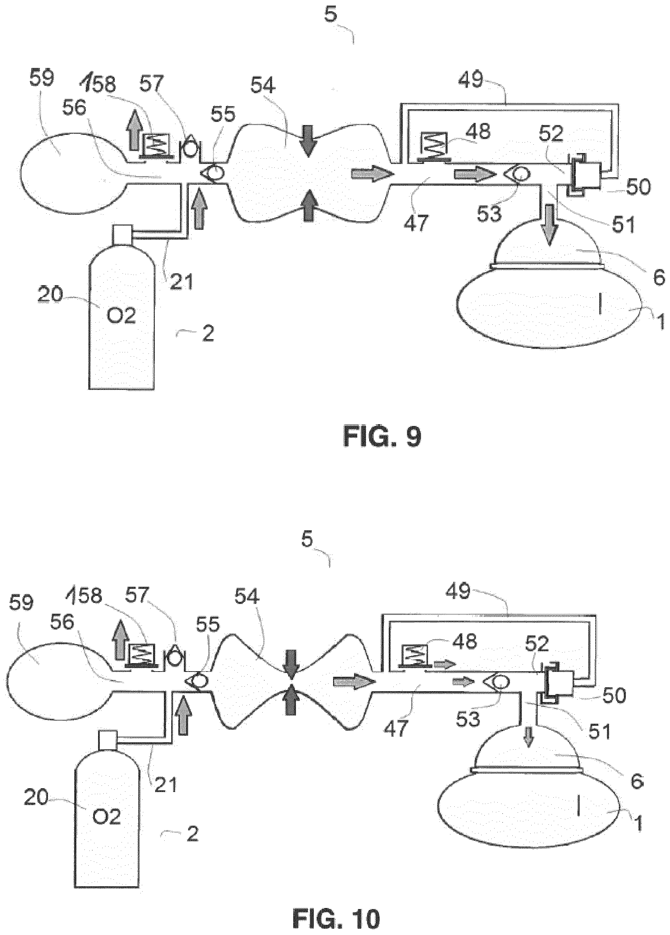

In Figure 9, the operator starts an insufflation by squeezing the flexible bag **54,** which will in turn open the third one-way valve **53.** By the same mechanism, the pressure across the pneumatic control valve **50,** which is between derivation conduct **49** and therefore chamber **50f,** and conduit **52** and therefore inlet **50a** will be close to 0. As a result, the pneumatic control valve **50** will remain closed, although the insufflation will create an increase in pressure in both sides of the pneumatic control valve **50.** As a consequence, all the gas exiting the bag **54** will travel into conduits **47** and **51** and be delivered to the patient **1,** via interface **6.**

On the other end of the bag, such positive pressure in the flexible bag **54** will force second one-way valve **55** to close and the oxygen coming from tubing **21** and entering conduit **56** will either fill the reservoir **59** or vent to the atmosphere through the first PEP exhaust valve **158,** whenever the pressure is greater than 5 cmH₂O.

At some point during the insufflations, the pressure may become too elevated. The resuscitation bag of the present invention provides a means to control this pressure as shown in Figure 10. This function is made possible by PPEAK valve **48** which is similar to the first PEP exhaust valve **158,** although its load spring is set in a way that only a pressure greater than 20 cm H₂O, for example, opens it and limits the pressure into conduits **47, 51** and patient's airways at this set value.

During the insufflation described with reference to Figures 9 and 10, the pneumatic control valve **50** (as shown in Fig. 13A & 13B) assists the operator. Indeed, in case of a thoracic compression the pressure on the patient **1** side will increase, for instance above 20 cmH₂O if we consider the compression occurred while PPEAK valve **48** was limiting the pressure, and close the third one-way valve **53.** This will create an imbalance in terms of pressure between conduits **51, 52** and inlet **50a** and their counterpart, e.g. conduits **47,** derivation conduct **49** and chamber **50f.** As soon as this imbalance exceeds the opening pressure, causing a differential pressure of 5 mm H₂O, the pneumatic control valve **50** will open and make a fluidic connection between inlet **50a** and outlet **50c,** allowing the volume expelled by the patient **1** to travel through interface **6,** conduits **51** and **52,** inlet **50a** and exhaust port, or outlet **50c.**

Figure 11 shows the expiration phase, when the operator has stopped squeezing the bag **54,** which enters an expansion phase. This creates a negative pressure which will open the second one-way valve **55,** which will in turn: i) direct flow from tubing **21** into bag **54** via the first conduit element **56;** ii) empty reservoir **59** into bag **54** via first conduit element **56;** and iii) open one-way admission valve **57** which will let ambient air flow into bag **54** via conduit **56.**

The same negative pressure will hold back the third one-way valve **53,** close overpressure or PPEAK valve **48** and decrease the pressure in derivation conduct **49,** which will in turn dramatically decrease the pressure in chamber **50f** of pneumatic control valve **50.** As the pressure in the patient's airways is high as a consequence of the past insufflation, the pneumatic control valve **50** opens to make a fluidic connection between inlet **50a** and outlet **50c,** allowing the volume expired by the patient **1** to travel through interface **6,** conduits **51** and **52,** inlet **50a** and exhaust port, or outlet **50c.** The pneumatic control valve **50** will remain open until an equilibrium is met between pressures in conduits **47** and **51,** which, by virtue of the description above, should be around the pressure set by the first PEP exhaust valve **158,** e.g. 5 cm H₂O and the patient **1** has returned to a low pressure level where subsequent thoracic compressions can occur, as described with reference to Figure 7A.

The resuscitation bag **5** of the present invention has the ability to allow safe insufflations by limiting the pressure at the patient's airways while authorizing compression phases, therefore optimizing hemodynamic of the patient, and to further apply a positive pressure in the patient's airways during the thoracic decompressions to help keep the lung alveoli of the patient open and further enhance gas exchange.

A second embodiment of the resuscitation bag **5,** not according to the present invention, that further enhances TCs, is shown in Figure 12.

Following a TC , as shown in Figure 7, the gas flowing into the patient **1,** during the thoracic decompression phase as illustrated in Figure 8, will partly be composed of the gas expelled from the patient **1** during TC and present in the interface **6** and conduits **51** and **52.**

This gas contains a "high" level of CO₂, which replaces valuable oxygen and further prevents the CO₂ clearance from the lung.

In many cases, it is advantageous:
- to lower as much as possible the space in which the CO₂ can be present, e.g. interface **6** and conduits **51** and **52,** and
- to "flush" out a maximum of CO₂ rich-gases, over the course of the resuscitation process.

In this aim, according to the second embodiment shown in Figure 12, the pneumatic control valve **50** is arranged directly in the region of the interface **6** so as to be fluidly connected to interface **6** via conduit **52.** Thus, pneumatic control valve **50** can more efficiently vent CO₂-enriched gases exhaled by patient **1** to the atmosphere, thereby avoiding CO₂ build-up into conduit **51.** Further, an oxygen distribution system **8** comprising a by-pass line **83** and a gas distributor **81** is provided. The by-pass line **83** is arranged between the gas distributor **81** fed by the oxygen source **2** and the interface **6.**

The inlet of the gas distributor **81** is fluidly connected to the oxygen source **2** via oxygen line or tubing **21.** In other words, the gas distributor **81** is arranged on the oxygen line **21.**

The distributor **81,** when manually operated by the operator, diverts a portion of the total incoming oxygen flow either to the downstream portion **82** of the oxygen line **21,** that is connected to resuscitation bag **5** via the first conduit element **56,** or to the by-pass line **83** that is fluidly connected to the interface **6** via an admission port **84.**

By acting on gas distributor **81,** e.g. a proportional diverting valve, the operator can select/allocate the respective amounts of oxygen flowing into by-pass tubing **83** and further into the downstream portion **82** of the oxygen line **21.** The first oxygen flow conveyed by the downstream portion **82** of the oxygen line **21** enters into the first conduit element **56** and, as already explained (cf. Fig. 7 and 8), when no gas insufflation is performed, helps keep a pressure of 5 cm H₂O in the flexible bag **54** and subsequent conduit **47,** derivation conduct **49** and chamber **50f,** thanks to the first PEP exhaust valve **158.**

Further, the second oxygen flow conveyed by the by-pass tubing **83** enters into interface **6,** such as a respiratory mask, via the admission port **84.** As the oxygen flow is continuous, a pressure build-up occurs in interface **6** and conduit **52** and further a pressure imbalance across pneumatic control valve **50** makes the fluidic connection between inlet conduit **50a** and outlet conduit **50c** to vent to the atmosphere, excessive flow, as hereinabove described in connection with Figure 7 to 11. Such a gas venting will also drag to the atmosphere any residual CO₂ from interface **6** and conduit **52.** Vented CO₂ is substituted by fresh oxygen delivered by by-pass line **83.**

The resuscitation bag **5** of the present invention constitutes a great improvement over those of the prior art.

## Claims

1. An artificial resuscitation bag (5) comprising:
- a deformable bag (54) comprising a gas inlet (54A) and a gas outlet (54B),
- a gas reservoir (59) comprising an outlet orifice (59A),
- a first conduit element (56) fluidly connected to the outlet orifice (59A) of the gas reservoir (59) and to the gas inlet (54A) of the deformable bag (54),
- a first one-way admission valve (57) arranged in the first conduit element (56) and fluidly communicating with the ambient atmosphere for allowing ambient air to enter into the first conduit element (56), and
- a second one-way valve (55) arranged in the first conduit element (56) between the first one-way admission valve (57) and the gas inlet (54A) of the deformable bag (54) for allowing gas to travel only from the first conduit element (56) to the deformable bag (54),
and further comprising :
- a first PEP exhaust valve (158) arranged in the first conduit element (56) and fluidly communicating with the ambient atmosphere for venting gas to the atmosphere when the gas pressure, into the first conduit element (56), exceeds a given threshold, and
- a gas conduit (47) arranged in fluid communication with the gas outlet (54B) of the deformable bag (54),
- an overpressure valve (48) arranged in the gas conduit (47),
- a third one-way valve (53) arranged in said gas conduit (47) downstream of the overpressure valve (48), and
- a gas delivery conduit (51) in fluid communication with the gas conduct (47) for conveying at least part of the gas circulating into the gas conduct (47) to a patient interface (6),
**characterized in that** the first PEP exhaust valve (158) comprises a spring (158d) and a membrane (158b), said spring (158d) applying a constant force on the membrane (158b) corresponding to the threshold pressure, the opening pressure of said PEP exhaust valve (158) being of between 1 cmH₂O and 30 cmH₂O,
and **in that** the artificial resuscitation bag (5) further comprises :
- a pneumatic control valve (50) arranged in the gas conduit (47) downstream of the third one-way valve (48), and
- a derivation conduct (49) having a first end (49A) fluidly connected to the gas conduit (47) between the gas outlet (54B) of the deformable bag (54) and the overpressure valve (48), and a second end (49B) fluidly connected to the pneumatic control valve (50), the pneumatic control valve (50) further comprising an inner compartment (50f), the second end (49B) of the derivation conduct (49) being fluidly connected to said inner compartment (50f) of the pneumatic control valve (50).

2. The artificial resuscitation bag of claim 1, wherein the opening pressure of the PEP exhaust valve (158) is of at least 5 cmH₂O.

3. The artificial resuscitation bag of claim 1, wherein the pneumatic control valve (50) comprises a deformable membrane (50b).

4. The artificial resuscitation bag of claim 1, wherein the third one-way valve (53) is configured for allowing a circulation of gas in the gas conduit (47) only in the direction from the deformable bag (54) toward the pneumatic control valve (50).

5. The artificial resuscitation bag of claim 1, wherein the first PEP exhaust valve (158) comprises an inlet port (158a) in fluid communication with the first conduit element (56).

6. The artificial resuscitation bag of claim 1, wherein the first conduit element (56) comprises an oxygen entry (56A) arranged between the outlet orifice (59A) of the gas reservoir (59) and the second one-way valve (55).

7. An installation for resuscitating a person in state of cardiac arrest comprising:
- an artificial resuscitation bag according to claim 1, and
- an O₂ source fluidly connected to the artificial resuscitation bag by means of an oxygen line, for providing oxygen to said artificial resuscitation bag.

## Patentansprüche

1. Künstlicher Beatmungsbeutel (5), umfassend:
- einen verformbaren Beutel (54), der einen Gaseinlass (54A) und einen Gasauslass (54B) umfasst,
- einen Gasbehälter (59), der eine Auslassöffnung (59A) umfasst,
- ein erstes Kanalelement (56), das fluidisch mit der Auslassöffnung (59A) des Gasbehälters (59) und mit dem Gaseinlass (54A) des verformbaren Beutels (54) verbunden ist,
- ein erstes Einweg-Einlassventil (57), das in dem ersten Kanalelement (56) angeordnet und fluidisch mit der Umgebungsatmosphäre verbunden ist, um zu ermöglichen, dass Umgebungsluft in das erste Kanalelement (56) eintritt, und
- ein zweites Einwegventil (55), das in dem ersten Kanalelement (56) zwischen dem ersten Einweg-Einlassventil (57) und dem Gaseinlass (54A) des verformbaren Beutels (54) angeordnet ist, um zu ermöglichen, dass sich Gas nur von dem ersten Kanalelement (56) zum verformbaren Beutel (54) bewegt,
und ferner umfassend:
- ein erstes PEP-Auslassventil (158), das in dem ersten Kanalelement (56) angeordnet und fluidisch mit der Umgebungsatmosphäre verbunden ist, um Gas an die Atmosphäre abzulassen, wenn der Gasdruck in dem ersten Kanalelement (56) einen gegebenen Schwellenwert übersteigt, und
- einen Gaskanal (47), der in Fluidverbindung mit dem Gasauslass (54B) des verformbaren Beutels (54) angeordnet ist,
- ein Überdruckventil (48), das in dem Gaskanal (47) angeordnet ist,
- ein drittes Einwegventil (53), das in dem Gaskanal (47) dem Überdruckventil (48) nachgelagert angeordnet ist, und
- einen Gaszufuhrkanal (51) in Fluidverbindung mit dem Gaskanal (47) zum Transportieren von mindestens einem Teil des Gases, das in dem Gaskanal (47) zirkuliert, an eine Patientenschnittstelle (6),
**dadurch gekennzeichnet, dass** das erste PEP-Auslassventil (158) eine Feder (158d) und eine Membran (158b) umfasst, wobei die Feder (158d) eine konstante Kraft auf die Membran (158b) aufbringt, die dem Schwellendruck entspricht, wobei der Öffnungsdruck des PEP-Auslassventils (158) zwischen 1 cmH₂O und 30 cmH₂O beträgt,
und dadurch, dass der künstliche Beatmungsbeutel (5) ferner umfasst:
- ein pneumatisches Steuerventil (50), das in dem Gaskanal (47) dem dritten Einwegventil (48) vorgelagert angeordnet ist, und
- einen Abzweigungskanal (49) mit einem ersten Ende (49A), das fluidisch mit dem Gaskanal (47) zwischen dem Gasauslass (54B) des verformbaren Beutels (54) und dem Überdruckventil (48) verbunden ist, und einem zweiten Ende (49b), das fluidisch mit dem pneumatischen Steuerventil (50) verbunden ist, wobei das pneumatische Steuerventil (50) ferner einen Innenraum (50f) umfasst, wobei das zweite Ende (49B) des Abzweigungskanals (49) fluidisch mit dem Innenfach (50f) des pneumatischen Steuerventils (50) verbunden ist.

2. Künstlicher Beatmungsbeutel nach Anspruch 1, wobei der Öffnungsdruck des PEP-Auslassventils (158) mindestens 5 cmH₂O beträgt.

3. Künstlicher Beatmungsbeutel nach Anspruch 1, wobei das pneumatische Steuerventil (50) eine verformbare Membran (50b) umfasst.

4. Künstlicher Beatmungsbeutel nach Anspruch 1, wobei das dritte Einwegventil (53) dazu ausgelegt ist, eine Zirkulation des Gases in dem Gaskanal (47) nur in der Richtung von dem verformbaren Beutel (54) hin zu dem pneumatischen Steuerventil (50) zu erlauben.

5. Künstlicher Beatmungsbeutel nach Anspruch 1, wobei das erste PEP-Auslassventil (158) einen ersten Einlassanschluss (158a) in Fluidverbindung mit dem ersten Kanalelement (56) umfasst.

6. Künstlicher Beatmungsbeutel nach Anspruch 1, wobei das erste Kanalelement (56) einen Sauerstoffeingang (56A) umfasst, der zwischen der Auslassöffnung (59A) des Gasbehälters (59) und dem zweiten Einwegventil (55) angeordnet ist.

7. Installation zur Wiederbelebung einer Person in einem Zustand des Herzstillstands, umfassend:
- einen künstlichen Beatmungsbeutel nach Anspruch 1 und
- eine O₂-Quelle, die mithilfe einer Sauerstoffleitung fluidisch mit dem künstlichen Beatmungsbeutel verbunden ist, um Sauerstoff für den künstlichen Beatmungsbeutel bereitzustellen.

## Revendications

1. Sac de réanimation artificielle (5) comprenant :
- un sac déformable (54) comprenant une entrée de gaz (54A) et une sortie de gaz (54B),
- un réservoir de gaz (59) comprenant un orifice de sortie (59A),
- un premier élément de conduit (56) relié fluidiquement à l'orifice de sortie (59A) du réservoir de gaz (59) et à l'entrée de gaz (54A) de la poche déformable (54),
- une première soupape d'admission unidirectionnelle (57) agencée dans le premier élément de conduit (56) et communiquant fluidiquement avec l'atmosphère ambiante pour permettre à l'air ambiant d'entrer dans le premier élément de conduit (56), et
- une deuxième soupape unidirectionnelle (55) agencée dans le premier élément de conduit (56) entre la première soupape d'admission unidirectionnelle (57) et l'entrée de gaz (54A) du sac déformable (54) pour permettre au gaz de se déplacer uniquement du premier élément de conduit (56) au sac déformable (54),
et comprenant en outre :
une première soupape d'échappement PEP (158) agencée dans le premier élément de conduit (56) et communiquant de manière fluide avec l'atmosphère ambiante pour évacuer le gaz vers l'atmosphère lorsque la pression du gaz, dans le premier élément de conduit (56), dépasse un seuil donné, et
- un conduit de gaz (47) agencé en communication fluidique avec la sortie de gaz (54B) du sac déformable (54),
- une soupape de surpression (48) agencée dans le conduit de gaz (47),
- une troisième soupape unidirectionnelle (53) agencée dans ledit conduit de gaz (47) en aval de la soupape de surpression (48), et
- un conduit d'alimentation en gaz (51) en communication fluidique avec le conduit de gaz (47) pour acheminer au moins une partie du gaz circulant dans le conduit de gaz (47) à une interface patient (6),
**caractérisé en ce que** la première soupape d'échappement PEP (158) comprend un ressort (158d) et une membrane (158b), ledit ressort (158d) appliquant une force constante sur la membrane (158b) correspondant à la pression de seuil, la pression d'ouverture de ladite soupape d'échappement PEP (158) étant comprise entre 1 cmH₂O et 30 cmH₂O,
et **en ce que** le sac de réanimation artificielle (5) comprend en outre :
- une vanne de commande pneumatique (50) agencée dans le conduit de gaz (47) en aval de la troisième soupape unidirectionnelle (48), et
- un conduit de dérivation (49) ayant une première extrémité (49A) reliée fluidiquement au conduit de gaz (47) entre la sortie de gaz (54B) du sac déformable (54) et la soupape de surpression (48), et une seconde extrémité (49B) reliée fluidiquement à la vanne de commande pneumatique (50), la vanne de commande pneumatique (50) comprenant en outre un compartiment intérieur (50f), la seconde extrémité (49B) du conduit de dérivation (49) étant reliée fluidiquement audit compartiment intérieur (50f) de la vanne de commande pneumatique (50).

2. Sac de réanimation artificielle selon la revendication 1, la pression d'ouverture de la soupape d'échappement PEP (158) étant d'au moins 5 cmH₂O.

3. Sac de réanimation artificielle selon la revendication 1, la vanne de commande pneumatique (50) comprenant une membrane déformable (50b).

4. Sac de réanimation artificielle selon la revendication 1, la troisième soupape unidirectionnelle (53) étant configurée pour permettre une circulation de gaz dans le conduit de gaz (47) uniquement dans la direction allant du sac déformable (54) vers la vanne de commande pneumatique (50).

5. Sac de réanimation artificielle selon la revendication 1, la première soupape d'échappement PEP (158) comprenant un orifice d'entrée (158a) en communication fluidique avec le premier élément de conduit (56).

6. Sac de réanimation artificielle selon la revendication 1, le premier élément de conduit (56) comprenant une entrée d'oxygène (56A) agencée entre l'orifice de sortie (59A) du réservoir de gaz (59) et la deuxième soupape unidirectionnelle (55).

7. Installation de réanimation d'une personne en état d'arrêt cardiaque comprenant :
- un sac de réanimation artificielle selon la revendication 1, et
- une source d'O₂ reliée fluidiquement au sac de réanimation artificielle au moyen d'une ligne d'oxygène, pour fournir de l'oxygène audit sac de réanimation artificielle.
